# EUROPÄISCHE PATENTSCHRIFT

(11) **EP 1 567 908 B1**
(45) Veröffentlichungstag und Bekanntmachung des Hinweises auf die Patenterteilung: **19.04.2006**
(21) Anmeldenummer: 03782251.7
(22) Anmeldetag: 28.11.2003
(51) Int. Cl.: G02C 7/08, A61B 3/10, A61B 3/103

(54) **SELBSTFOKUSSIERENDE BRILLE UND VERFAHREN ZUM SELBSTFOKUSSIEREN EINER BRILLE**
AUTO-FOCUSSING GLASSES AND METHOD FOR AUTO-FOCUSSING A PAIR OF GLASSES
LUNETTES A MISE AU POINT AUTOMATIQUE ET PROCEDE DE MISE AU POINT AUTOMATIQUE DE LUNETTES

(30) Priorität: 06.12.2002 DE 10258729
(43) Veröffentlichungstag der Anmeldung: 31.08.2005
(73) Patentinhaber: Carl Zeiss Vision GmbH, 73430 Aalen (DE)
(72) Erfinder: WAHL, Eberhard, 73430 Aalen (DE); SCHNITZER, Peter, 89522 Heidenheim (DE); SCHÖTTLE, Klaus-Peter, 73447 Oberkochen (DE); KOHSCHMIEDER, Ingo, 07743 Jena (DE)
(74) Vertreter: Witte, Alexander
(86) Internationale Anmeldenummer: PCT/EP2003/013462
(87) Internationale Veröffentlichungsnummer: WO 2004/053569

(56) Entgegenhaltungen:
- US-A- 4 300 818
- US-A- 4 756 605
- US-A- 5 359 444

## Beschreibung

Die Erfindung betrifft eine selbstfokussierende Brille mit mindestens einem in seiner Brechkraft einstellbaren Brillenglas, und mit Mitteln zum Einstellen der Brechkraft des Brillenglases in Abhängigkeit von einem gemessenen, den Akkommodationszustand wiedergebenden Parameter eines zugehörigen Auges eines Benutzers der Brille.

Die Erfindung betrifft ferner ein Verfahren zum Selbstfokussieren einer Brille mit mindestens einem in seiner Brechkraft einstellbaren Brillenglas und mit Mitteln zum Einstellen der Brechkraft des Brillenglases in Abhängigkeit von einem gemessenen, den Akkommodationszustand wiedergebenden Parameter eines zugehörigen Auges eines Benutzers der Brille.

Eine Brille und ein Verfahren der vorstehend genannten Art sind aus der US 4 300 818 A bekannt.

Wenn eine Person mit unbewaffnetem Auge Objekte in unterschiedlicher Entfernung fixieren möchte, ist es erforderlich, dass das Auge entsprechend der jeweiligen Entfernung des Objektes akkommodiert. Das selbe gilt, wenn die Person eine Brille trägt, deren Gläser eine einzige, fest eingestellte Brechkraft aufweisen. Das führt jedoch bei Personen mit eingeschränkter Akkommodationsfähigkeit zu Problemen, da diesen Personen ein Scharfsehen nur über einen begrenzten Entfernungsbereich möglich ist.

Wenn die Person hingegen eine Brille mit so genannten Gleitsichtgläsern trägt, die bereichsweise mit einer unterschiedlichen Brechkraft versehen sind, kann die Person durch Änderung der Sehrichtung bzw. Kopfhaltung das jeweilige Objekt mit einem Bereich des Gleitsichtglases anvisieren, dessen Brechkraft gerade zu der Entfernung dieses Objektes passt. Das Auge muss dann nicht mehr akkommodieren, weil ihm für jede Entfernung bereits ein Brillenglasbereich passender Brechkraft angeboten wird.

Darüber hinaus sind auch schon so genannte selbstfokussierende oder adaptive Brillen vorgeschlagen worden, deren Gläser insgesamt durch Fernsteuerung in ihrer Brechkraft einstellbar sind. Ein Benutzer kann dabei Objekte in unterschiedlicher Entfernung fixieren, ohne einen zugehörigen Bereich des Brillenglases auswählen zu müssen. Eine Akkommodation findet dabei ebenfalls nicht statt.

Für derartige Brillen sind Gläser erforderlich, die mittels elektrischer Signale in ihrer Brechkraft einstellbar sind.

Aus der US 4 756 605 ist eine derartige Brille bekannt, deren Gläser als Flüssigkristalle (LCD) ausgebildet sind. Mittels eines am Brillenbügel angeordneten Potentiometers kann bei dieser bekannten Brille die Brechkraft des rechten und des linken Brillenglases manuell und individuell eingestellt werden.

Nachteil dieser bekannten Brille ist, dass die Brechkraft der beiden Brillengläser in umständlicher Weise jeweils separat und manuell eingestellt werden muss. Bei wechselnder Blickrichtung kann somit die Scharfeinstellung der Brillengläser nur mit zeitlicher Verzögerung vorgenommen werden.

Aus der US 5 359 444 ist eine selbstfokussierende Brille bekannt, bei der die vorstehend genannten Nachteile vermieden werden. Diese bekannte Brille weist an dem Brillengestell zwei Infrarot-Entfernungsmesser auf, mit denen der Abstand zu dem fixierten Objekt gemessen wird. In Abhängigkeit von dem gemessenen Abstand wird dann die Brechkraft der beiden Brillengläser automatisch eingestellt.

Obwohl diese bekannte Brille damit den Nachteil einer manuellen Nachstellung vermeidet, ist diese Lösung immer noch in der Praxis mit erheblichen Nachteilen behaftet, insbesondere dann, wenn der Entfernungsmesser nicht exakt feststellen kann, welches Objekt im Gesichtsfeld nun fixiert werden soll. Dies trifft z.B. dann zu, wenn jemand z.B. durch ein in der Nähe befindliches Fenster nach außen blickt und der Entfernungsmesser dann nicht differenzieren kann, ob die Ebene des Fensterrahmens oder z.B. eine durch das Fenster sichtbare Landschaft fixiert werden sollen. Entsprechendes gilt dann, wenn jemand an einer Hauskante vorbei blickt oder versucht, ein bewegliches Objekt vor einem festen Hintergrund zu fixieren.

Aus der eingangs genannten US 4 300 818 ist eine weitere selbstfokussierende Brille bekannt, bei der zum Einstellen der Brechkraft der Brillengläser eine Messung an den Augen des Benutzers vorgenommen wird, um einen Parameter zu messen, der den Akkommodationszustand des Auges wiedergibt. Bei dieser bekannten Brille wird hierzu der so genannte Konvergenzwinkel gemessen, d.h. der Winkel, den die Achsen der beiden Augen miteinander einschließen. Zu diesem Zweck werden zwei Lichtstrahlen auf die Augäpfel des Benutzers gerichtet, und es wird detektiert, ob der entsprechende Reflektionspunkt auf der hellen Sclera oder der dunklen Cornea des Auges liegt. In Abhängigkeit von dem auf diese Weise bestimmten Konvergenzwinkel wird dann die Brechkraft der Brillengläser eingestellt.

Obwohl bei dieser bekannten selbstfokussierenden Brille somit auf den Blickwunsch des Benutzers abgestellt wird, ergeben sich immer noch praktische Nachteile.

Bei dieser bekannten Brille setzt nämlich die Erfassung des Konvergenzwinkels zwingend voraus, dass beide Augen des Benutzers vermessen werden. Es ist daher nicht möglich, jedes Auge individuell in seiner Brechkraft zu berücksichtigen.

Weiterhin setzt diese bekannte Brille voraus, dass es eine konstante Beziehung zwischen der Akkommodation des Auges und dem Konvergenzwinkel gibt, was in der Praxis durchaus nicht immer der Fall sein muss. Weiterhin ist die Messung dann nicht eindeutig, wenn der Benutzer die Augen in einer Vertikalrichtung bewegt. Darüber hinaus können sich im Regelkreis Instabilitäten einstellen, indem Schwingungen auftreten, wenn die Nachstellung der Brechkraft des Brillenglases und die Einstellung der optischen Achsen der Augen des Benutzers miteinander in Wechselwirkung treten.

Ein wesentlicher Nachteil der vorbekannten selbstfokussierenden Brillen ist jedoch, dass bei diesen Brillen eine Einstellung der Brechkraft der Brillengläser in einem Ausmaß vorgenommen wird, dass der Benutzer selbst das Auge überhaupt nicht mehr zu akkommodieren braucht. Damit erlahmt bei Personen mit nur eingeschränkter, in gewissem Umfang aber noch vorhandener Akkommodationsfähigkeit die Fähigkeit des Benutzers, eine Akkommodation durch seine eigenen Augen herzustellen, und die Akkommodationsfähigkeit wird innerhalb kürzester Zeit verlernt.

Der Erfindung liegt demgegenüber die Aufgabe zu Grunde, eine Brille sowie ein Verfahren der eingangs genannten Art dahingehend weiterzubilden, dass die vorstehend genannten Nachteile vermieden werden.

Insbesondere soll eine im Betrieb sichere und störunanfällige Brille bzw. ein zugehöriges Verfahren bereitgestellt werden. Weiterhin soll bewirkt werden, dass die noch vorhandene Fähigkeit des Benutzers zur Akkommodation seines Auges weiterhin trainiert wird und nicht verloren geht.

Bei einer Brille der eingangs genannten Art wird diese Aufgabe erfindungsgemäß dadurch gelöst, dass die Mittel die Brechkraft des Auges unmittelbar messen.

Bei einem Verfahren der eingangs genannten Art wird die der Erfindung zugrunde liegende Aufgabe dadurch gelöst, dass durch die Mittel die Brechkraft mindestens eines Auges unmittelbar gemessen wird.

Die der Erfindung zugrunde liegende Aufgabe wird auf diese Weise vollkommen gelöst.

Wenn nämlich die Brechkraft des Auges des Benutzers unmittelbar gemessen wird, so ist dies ein direktes Maß für die erforderliche kompensierende Brechkraft des zugehörigen Brillenglases. Anders als bei der Messung des Konvergenzwinkels muss daher nicht auf eine Hilfsabhängigkeit zwischen zwei ophthalmologischen Größen zurückgegriffen werden, weil die allein interessierende Größe, nämlich die Brechkraft, unmittelbar erfasst und verglichen wird.

Bei einer bevorzugten Ausgestaltung der erfindungsgemäßen Brille sind mindestens ein auf das Auge gerichteter Lichtsender und ein dem Lichtsender zugeordneter Lichtempfänger für ein von dem Auge reflektiertes Lichtsignal vorgesehen, wobei die Mittel die Brechkraft des Brillenglases in Abhängigkeit von dem reflektierten Lichtsignal einstellen.

Diese Maßnahme hat den Vorteil, dass mittels bewährter Methoden die Brechkraft des Auges unmittelbar gemessen werden kann. In bevorzugter Weise geschieht das mit nicht-sichtbarem Licht, insbesondere mit infrarotem Licht.

Bei einer bevorzugten Weiterbildung dieses Ausführungsbeispiels der Erfindung messen die Mittel die Brechkraft des mindestens einen Auges durch Einstrahlung eines Lichtstrahles in das Auge. Dies geschieht insbesondere dadurch, dass der Abstand zwischen einer Cornea und einer Vorderfläche einer Linse des Auges gemessen wird.

Diese Maßnahme hat den Vorteil, dass auf besonders einfache Weise die Brechkraft des Auges bestimmt werden kann. Andere Messmethoden sind jedoch im Rahmen der vorliegenden Erfindung ebenfalls einsetzbar.

Bei einer weiter bevorzugten Ausführungsform der erfindungsgemäßen Brille messen die Mittel die Brechkraft individuell für jedes Auge und stellen die Brechkraft des zu dem jeweiligen Auge gehörigen Brillenglases individuell ein.

Diese Maßnahme hat gegenüber dem referierten Stand der Technik den Vorteil, dass eine für jedes Auge individuelle Einstellung der Brechkraft des jeweils zugehörigen Brillenglases möglich ist.

Bei einer besonders bevorzugten Ausführungsform einer erfindungsgemäßen Brille stellen die Mittel die Brechkraft des mindestens einen Brillenglases erst nach Überschreiten eines vorbestimmten Grenzwertes der Brechkraft des zugehörigen Auges ein.

Diese Maßnahme hat den Vorteil, dass die natürliche Akkommodationsfähigkeit des Auges weiterhin trainiert wird, und zwar innerhalb ihrer (möglicherweise eingeschränkten) Grenzen. Solange nämlich das Auge des Benutzers nur innerhalb derjenigen Grenzen zur Akkommodation aufgefordert wird, die noch auf natürliche Weise bewirkt werden kann, greift die erfindungsgemäße Brille nicht ein. Erst wenn der vorbestimmte Grenzwert der Brechkraft des Auges erreicht wird, von dem ab eine natürliche Akkommodation bei dem jeweiligen Benutzer nicht mehr möglich ist, wird die Regelung der erfindungsgemäßen Brille aktiv.

Entsprechendes gilt für Ausführungsformen erfindungsgemäßer Verfahren.

Für die letztgenannte Variante ist besonders bevorzugt, wenn vorab die Grenze des vom Benutzer natürlich einstellbaren Brechkraftbereiches des mindestens einen Auges gemessen und der Grenzwert als vorbestimmter Anteil der Grenze, vorzugsweise als 80 bis 98 % der Grenze, vorgegeben wird.

Diese Maßnahme hat den Vorteil, dass in vorbestimmter Weise die Eingriffsschwelle für die erfindungsgemäße Regelung festgelegt werden kann, ohne dass jeweils die natürliche Grenze der Akkommodationsfähigkeit des Benutzers bestimmt werden muss.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: eine äußerst schematisierte Draufsicht auf ein Ausführungsbeispiel einer erfindungsgemäßen selbstfokussierenden Brille;
- Fig. 2: ein schematisiertes Flussdiagramm zur Erläuterung der Wirkungsweise der Brille gemäß Fig. 1.

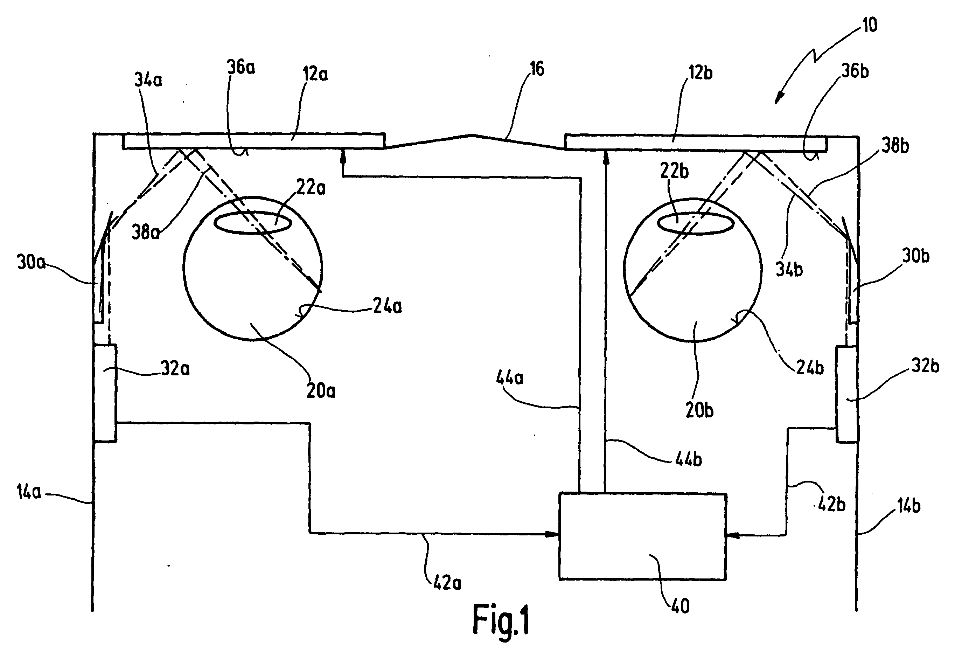

In Fig. 1 bezeichnet 10 insgesamt ein Ausführungsbeispiel einer erfindungsgemäßen selbstfokussierenden Brille. Die Brille 10 weist Gläser 12a, 12b auf, die durch Fernsteuerung in ihrer Brechkraft einstellbar sind. Derartige Brillengläser werden üblicherweise als Flüssigkristall- (LCD-) Elemente ausgebildet, wie z.B. aus der US 4 919 520, der US 5 066 301 und der US 4 601 545 bekannt ist.

Die Brille 10 verfügt ferner seitlich über Bügel 14a, 14b sowie einen die Gläser 12a, 12b verbindenden Steg 16.

Mit 20a, 20b sind in Fig. 1 die Augen eines Benutzers der Brille 10 angedeutet. In den Augen 20a, 20b sind jeweils die Linse 22a, 22b sowie die Netzhaut 24a, 24b eingezeichnet.

An den Bügeln 14a, 14b befinden sich Lichtsender 30a, 30b, die vorzugsweise nicht-sichtbares, insbesondere infrarotes Licht ausstrahlen. Außerdem sind an den Bügeln 14a, 14b Lichtempfänger 32a, 32b, beispielsweise Kameras, angeordnet.

Die Lichtsender 30a, 30b senden Lichtstrahlen 34a, 34b aus. Diese werden an hinteren Oberflächen 36a, 36b der Gläser 12a, 12b reflektiert und durch die Linsen 22a, 22b auf die Netzhäute 24a, 24b der Augen 20a, 20b geleitet. Die reflektierten Lichtstrahlen 38a, 38b werden von den Lichtempfängern 32a, 32b empfangen. Die daraus abgeleiteten Signale werden einem Steuergerät 40 über Steuerleitungen 42a, 42b zugeführt. Das Steuergerät 40 erzeugt aus diesen Eingangssignalen Stellsignale für die Gläser 12a, 12b und leitet diese den Gläsern 12a, 12b über weitere Steuerleitungen 44a, 44b zu.

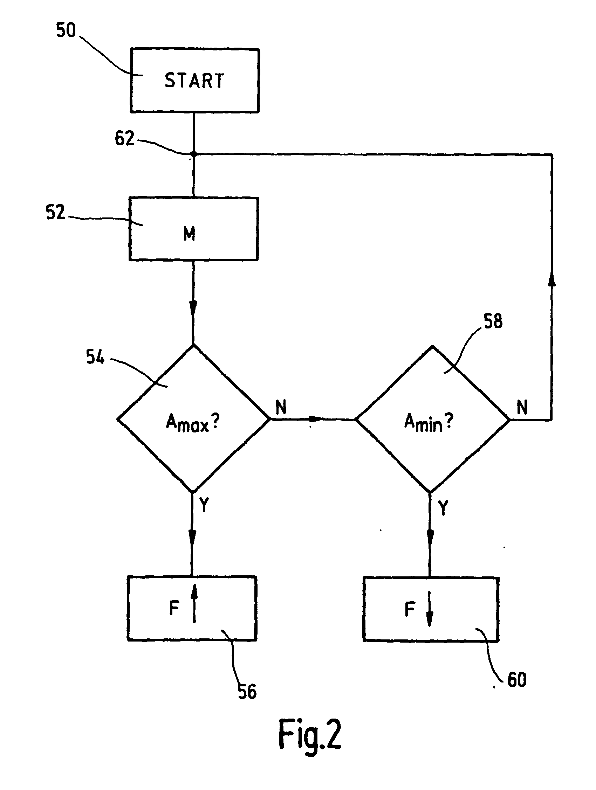

Die Funktionsweise der Brille 10 gemäß Fig. 1 soll nun an Hand des Flussdiagramms gemäß Fig. 2 näher erläutert werden.

In einem Block 50 ist angegeben, dass das erfindungsgemäße Verfahren zunächst gestartet wird.

Für jedes Auge 20a, 20b individuell wird nun in einem Block 52 eine Messung M der Brechkraft durchgeführt, indem die vorerwähnten Lichtstrahlen 34a, 34b bzw. 38a, 38b entsprechend ausgewertet werden.

Derartige Auswerteverfahren bzw. Messverfahren für die Brechkraft von Augen sind im Stand der Technik bekannt. So ist es z.B. möglich, den Abstand zwischen der Cornea und einer Vorderfläche der Linse 22a, 22b zu messen. Darüber hinaus sind auch Refraktionsmessungen möglich, wie sie z.B. in der US 4 834 528 sowie der DE 197 19 694 A1 beschrieben sind.

Das im Block 52 gemessene Signal, das der aktuellen Brechkraft des Auges 20a bzw. 20b entspricht, kann nun unmittelbar herangezogen werden, um die Brechkraft des jeweils zugehörigen Glases 12a bzw. 12b nachzustellen.

Erfindungsgemäß wird jedoch eine Vorgehensweise bevorzugt, bei der zunächst geprüft wird, ob ein Maximalwert der Akkommodation Aₘₐₓ bzw. ein Minimalwert Aₘᵢₙ des jeweiligen Auges 20a, 20b erreicht ist.

Im Flussdiagramm gemäß Fig. 2 wird in einem Block 54 zunächst geprüft, ob der Maximalwert Aₘₐₓ bzw. ein bestimmter Bruchteil, z.B. im Bereich zwischen 80 und 98 % des Maximalwertes Aₘₐₓ, erreicht ist. Nur wenn dies der Fall ist, wird in einem Block 56 die Brechkraft des zugehörigen Glases 12a bzw. 12b erhöht.

Wenn der Maximalwert Aₘₐₓ nicht erreicht ist, wird in einem Block 58 geprüft, ob ein Minimalwert Aₘᵢₙ erreicht wurde. Ist dies der Fall, wird in einem Block 60 die Brechkraft des zugehörigen Glases 12a bzw. 12b vermindert. Wenn auch der Minimalwert Aₘᵢₙ nicht erreicht wurde, ist dies ein Maß dafür, dass sich die Brechkraft des Auges 20a bzw. 20b noch innerhalb desjenigen Bereiches befindet, der vom Benutzer auf natürliche Weise eingestellt werden kann. In diesem Fall bleibt die erfindungsgemäße Regelung inaktiv, damit der Benutzer die Fähigkeit seines Auges 20a bzw. 20b zur natürlichen Akkommodation weiter nutzen und trainieren kann. Das Verfahren kehrt in diesem Falle an einem Punkt 62 wieder zum Ausgang zurück, um anschließend erneut im Block 52 eine Messung M vorzunehmen, weil der Benutzer inzwischen die Blickrichtung und damit die Fixierung eines Objektes geändert haben könnte.

Das in Fig. 2 dargestellte Flussdiagramm kann in schneller zeitlicher Folge durchlaufen werden, um eventuellen Änderungen der Anforderungen rasch folgen zu können.

## Patentansprüche

1. Selbstfokussierende Brille mit mindestens einem in seiner Brechkraft einstellbaren Brillenglas (12a, 12b), und mit Mitteln (40 - 44) zum Einstellen der Brechkraft des Brillenglases (12a, 12b) in Abhängigkeit von einem gemessenen, den Akkommodationszustand wiedergebenden Parameter eines zugehörigen Auges (20a, 20b) eines Benutzers der Brille (10), **dadurch gekennzeichnet, dass** die Mittel (40 - 44) die Brechkraft des Auges (20a, 20b) unmittelbar messen.

2. Brille nach Anspruch 1, **dadurch gekennzeichnet, dass** mindestens ein auf das Auge (20a, 20b) gerichteter Lichtsender (30a, 30b) und ein dem Lichtsender (30a, 30b) zugeordneter Lichtempfänger (32a, 32b) für ein von dem Auge (20a, 20b) reflektiertes Lichtsignal (38a, 38b) vorgesehen sind, und dass die Mittel (40 - 44) die Brechkraft des Brillenglases (12a, 12b) in Abhängigkeit von dem reflektierten Lichtsignal (38a, 38b) einstellen.

3. Brille nach Anspruch 2, **dadurch gekennzeichnet, dass** die Mittel (40 - 44) die Brechkraft des mindestens einen Auges (20a, 20b) durch Einstrahlung eines Lichtstrahles (34a, 34b) in das Auge (20a, 20b) messen.

4. Brille nach Anspruch 3, **dadurch gekennzeichnet, dass** die Mittel (40 - 44) den Abstand zwischen einer Cornea und einer Vorderfläche einer Linse (22a, 22b) des Auges (20a, 20b) messen.

5. Brille nach einem oder mehreren der Ansprüche 1 bis 4, **dadurch gekennzeichnet, dass** die Mittel (40 - 44) die Brechkraft individuell für jedes Auge (20a, 20b) messen und die Brechkraft des zu dem jeweiligen Auge gehörigen Brillenglases (12a, 12b) individuell einstellen.

6. Brille nach einem oder mehreren der Ansprüche 1 bis 5, **dadurch gekennzeichnet, dass** die Mittel (40 - 44) die Brechkraft des mindestens einen Brillenglases (12a, 12b) erst nach Überschreiten eines vorbestimmten Grenzwertes der Brechkraft des zugehörigen Auges (12a, 12b) einstellen.

7. Verfahren zum Selbstfokussieren einer Brille (10) mit mindestens einem in seiner Brechkraft einstellbaren Brillenglas (12a, 12b) und mit Mitteln (40 - 44) zum Einstellen der Brechkraft des Brillenglases (12a, 12b) in Abhängigkeit von einem gemessenen, den Akkommodationszustand wiedergebenden Parameter eines zugehörigen Auges (20a, 20b) eines Benutzers der Brille (10), **dadurch gekennzeichnet, dass** durch die Mittel (40 - 44) die Brechkraft mindestens eines Auges (20a, 20b) unmittelbar gemessen wird.

8. Verfahren nach Anspruch 7, **dadurch gekennzeichnet, dass** die Brechkraft durch Vermessen eines Abstandes zwischen einer Cornea und einer Vorderfläche einer Linse (22a, 22b) des Auges (20a, 20b) gemessen wird.

9. Verfahren nach Anspruch 7 oder 8, **dadurch gekennzeichnet, dass** die Brechkraft individuell für jedes Auge (20a, 20b) gemessen und die Brechkraft des zu dem jeweiligen Auge gehörigen Brillenglases (12a, 12b) individuell eingestellt wird.

10. Verfahren nach einem oder mehreren der Ansprüche 7 bis 9, **dadurch gekennzeichnet, dass** die Brechkraft des mindestens einen Brillenglases (12a, 12b) erst nach Überschreiten eines vorbestimmten Grenzwertes der Brechkraft des zugehörigen Auges (12a, 12b) eingestellt wird.

11. Verfahren nach Anspruch 10, **dadurch gekennzeichnet, dass** vorab die Grenze des vom Benutzer natürlich einstellbaren Brechkraftbereiches des mindestens einen Auges (20a, 20b) gemessen und der Grenzwert als vorbestimmter Anteil der Grenze, vorzugsweise als 80 - 98 % der Grenze, vorgegeben wird.

## Revendications

1. Lunettes à mise au point automatique avec au moins un verre de lunettes (12a, 12b) dont la puissance réfringente est réglable, et avec des moyens (40 - 44) pour le réglage de la puissance réfringente du verre de lunettes (12a, 12b) en fonction d'un paramètre mesuré reproduisant l'état d'accommodation d'un oeil correspondant (20a, 20b) d'un utilisateur des lunettes (10), **caractérisées en ce que** les moyens (40 - 44) mesurent directement la puissance réfringente de l'oeil (20a, 20b).

2. Lunettes selon la revendication 1, **caractérisées en ce qu'**il est prévu au moins un émetteur de lumière (30a, 30b) dirigé vers l'oeil (20a, 20b) et un récepteur de lumière (32a, 32b) associé à l'émetteur de lumière (30a, 30b) pour un signal lumineux (38a, 38b) réfléchi par l'oeil (20a, 20b), et **en ce que** les moyens (40 - 44) règlent la puissance réfringente du verre de lunettes (12a, 12b) en fonction du signal lumineux (38a, 38b) réfléchi.

3. Lunettes selon la revendication 2, **caractérisées en ce que** les moyens (40 - 44) mesurent la puissance réfringente d'au moins un oeil (20a, 20b) par la radiation d'un rayon lumineux (34a, 34b) dans l'oeil (20a, 20b).

4. Lunettes selon la revendication 3, **caractérisées en ce que** les moyens (40 - 44) mesurent la distance entre une cornée et une surface avant d'une lentille (22a, 22b) de l'oeil (20a, 20b).

5. Lunettes selon une ou plusieurs des revendications 1 à 4, **caractérisées en ce que** les moyens (40 - 44) mesurent la puissance réfringente individuellement pour chaque oeil (20a, 20b) et règlent individuellement la puissance réfringente du verre de lunettes (12a, 12b) correspondant à chaque oeil.

6. Lunettes selon une ou plusieurs des revendications 1 à 5, **caractérisées en ce que** les moyens (40 - 44) règlent la puissance réfringente d'au moins un verre de lunettes (12a, 12b) seulement après le dépassement d'une valeur limite prédéterminée de la puissance réfringente de l'oeil correspondant (12a, 12b).

7. Procédé pour la mise au point automatique de lunettes (10) avec au moins un verre de lunettes (12a, 12b) dont la puissance réfringente est réglable, et avec des moyens (40 - 44) pour le réglage de la puissance réfringente du verre de lunettes (12a, 12b) en fonction d'un paramètre mesuré reproduisant l'état d'accommodation d'un oeil correspondant (20a, 20b) d'un utilisateur des lunettes (10), **caractérisé en ce que** la puissance réfringente d'au moins un oeil (20a, 20b) est mesurée directement par tes moyens (40-44).

8. Procédé selon la revendication 7, **caractérisé en ce que** la puissance réfringente est mesurée à l'aide de la mesure d'une distance entre une cornée et une surface avant d'une lentille (22a, 22b) de l'oeil (20a, 20b).

9. Procédé selon la revendication 7 ou 8, **caractérisé en ce que** la puissance réfringente est mesurée individuellement pour chaque oeil (20a, 20b) et la puissance réfringente du verre de lunettes (12a, 12b) correspondant à chaque oeil est réglée individuellement.

10. Procédé selon une ou plusieurs des revendications 7 à 9, **caractérisé en ce que** la puissance réfringente d'au moins un verre de lunettes (12a, 12b) est réglée seulement après le dépassement d'une valeur limite prédéterminée de la puissance réfringente de l'oeil correspondant (12a, 12b).

11. Procédé selon la revendication 10, **caractérisé en ce que** la limite de la plage de puissance réfringente réglable naturellement par l'utilisateur d'au moins un oeil (20a, 20b) est mesurée au préalable et la valeur limite est prédéterminée comme pourcentage prédéterminé de la limite, de préférence 80% - 98% de la limite.

## Claims

1. A self-focussing spectacle comprising at least one spectacle lens (12a, 12b) being adapted to be adjusted in its refracting power, and means (40 - 44) for adjusting the refracting power of the spectacle lens (12a, 12b) as a function of a measured parameter representing the state of accommodation of a corresponding eye (20a, 20b) of a user of the spectacle (10), **characterized in that** the means (40 - 44) measure the refractive power of the eye (20a, 20b) directly.

2. The spectacle of claim 1, **characterized in that** at least one light transmitter (30a, 30b) directed to the eye (20a, 20b), as well as a light receiver (32a, 32b) associated to the light transmitter (30a, 30b) for receiving a light signal (38a, 38b) reflected by the eye (20a, 20b) are provided, and that the means (40 - 44) adjust the refractive power of the spectacle lens (12a, 12b) as a function of the reflected light signal (38a, 38b).

3. The spectacle of claim 2, **characterized in that** the means (40 - 44) measure the refractive power of the at least one eye (20a, 20b) by irradiating a light beam (34a, 34b) into the eye (20a, 20b).

4. The spectacle of claim 3, **characterized in that** the means (40 - 44) measure the distance between a cornea and a front surface of a lens (22a, 22b) of the eye (20a, 20b).

5. The spectacle of one or more of claims 1 to 4, **characterized in that** the means (40 - 44) measure the refractive power individually for each eye (20a, 20b), and individually adjust the refractive power of the spectacle lens (12a, 12b) corresponding to the respective eye.

6. The spectacle of one or more of claims 1 to 5, **characterized in that** the means (40 - 44) adjust the refractive power of the at least one spectacle lens (12a, 12b) only when a predetermined threshold value of the refractive power of the corresponding eye (12a, 12b) has been exceeded.

7. A method for self-focussing a spectacle (10) comprising at least one spectacle lens (12a, 12b) being adapted to be adjusted in its refracting power, and means (40 - 44) for adjusting the refracting power of the spectacle lens (12a, 12b) as a function of a measured parameter representing the state of accommodation of a corresponding eye (20a, 20b) of a user of the spectacle (10), **characterized in that** the refractive power of the eye (20a, 20b) is directly measured by the means (40 - 44).

8. The method of claim 7, **characterized in that** the refractive power is measured by measuring a distance between a cornea and a front surface of a lens (22a, 22b) of the eye (20a, 20b).

9. The method of claim 7 or 8, **characterized in that** the refractive power is measured individually for each eye (20a, 20b) and that the refractive power of the spectacle lens (12a, 12b) corresponding to the respective eye (12a, 12b) is adjusted individually.

10. The method of one or more of claims 7 to 9, **characterized in that** the refractive power of the at least one spectacle lens (12a, 12b) is adjusted only when a predetermined threshold value of the refractive power of the corresponding eye (12a, 12b) has been exceeded.

11. The method of claim 10, **characterized in that** the limit of the refractive power range of the at least one eye (20a, 20b) that may be naturally adjusted by the user is measured at the outset, and that the threshold value is set to be a predetermined portion of that limit, preferably 80 to 98 % of the limit.
